(19)

Europäisches Patentamt
European Patent Office
Office européen des brevets

(11) **EP 4 327 744 A1**

(12) **EUROPEAN PATENT APPLICATION**
published in accordance with Art. 153(4) EPC

(43) Date of publication:
**28.02.2024 Bulletin 2024/09**

(21) Application number: **22791583.2**

(22) Date of filing: **31.03.2022**

(51) International Patent Classification (IPC):
*A61B 5/294* (2021.01)    *A61B 5/388* (2021.01)
*G06F 3/01* (2006.01)

(52) Cooperative Patent Classification (CPC):
**A61B 5/294; A61B 5/388; G06F 3/01**

(86) International application number:
**PCT/JP2022/016906**

(87) International publication number:
**WO 2022/224808 (27.10.2022 Gazette 2022/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.04.2021 JP 2021071933**

(71) Applicant: **Meltin MMI Co., Ltd.
Tokyo 1040033 (JP)**

(72) Inventors:
• **KASUYA, Masahiro
  Tokyo 104-0033 (JP)**
• **SEKI, Tatsuya
  Tokyo 104-0033 (JP)**

(74) Representative: **Uexküll & Stolberg
Partnerschaft von
Patent- und Rechtsanwälten mbB
Beselerstraße 4
22607 Hamburg (DE)**

(54) **METHOD, SYSTEM, AND ELECTRODE STRUCTURE FOR ACQUIRING ELECTRICAL SIGNALS FROM PLURALITY OF LEAD WIRES, AND METHOD FOR MANUFACTURING SAID ELECTRODE STRUCTURE**

(57)    This method for acquiring electrical signals from a plurality of lead wires uses an electrode structure to detect a plurality electrical signals from the plurality of lead wires, and includes: the feature wherein the electrode structure comprises a plurality of electrode layers arranged so as to be separated from each other, each of the plurality of electrode layers has a plurality of holes having dimensions such that the plurality of lead wires can pass therethrough, and each of the plurality of holes is connected to a conductor that conducts an electrical signal propagating through the lead wire passing through the hole to the outside of the electrode structure; and the feature of distinguishing between the detected plurality of electrical signals.

FIG.7

700

Detect a plurality of electrical signals from a plurality of lead wires by using an electrode structure — S701

Distinguish the plurality of detected electrical signals — S702

EP 4 327 744 A1

## Description

[Technical Field]

The present disclosure relates to a method, system, and electrode structure for obtaining electrical signals from a plurality of lead wires, and a method of manufacturing said electrode structure. More specifically, the present disclosure relates to a method, etc. for obtaining electrical signals from a plurality of nerves of an organism.

[Background Art]

Development of a technology known as brain-machine interface (BMI) is ongoing. BMI refers to a technology for directly connecting the brain of an organism with a machine.

Information directly obtained from the brain through BMI can be utilized in various applications. For example, an object is moved based on information directly obtained from the brain or information obtained from an object is inputted into the brain, whereby the object is moved as if the object is the organism's own body, and a new sensory organ can be obtained. The object can be an external instrument, the organism's own body, another organism's body, etc. An external instrument may be, for example, a body assisting apparatus such as a prosthetic arm or a prosthetic leg, or a remotely controllable robot. Another organism's body is, for example, another organism's brain, which can be connected to a similar BMI.

[Citation List]

[Patent Literature]

[PTL 1] International Publication No. WO 2018/147407

[Summary of Invention]

[Technical Problem]

The objective of the present invention is to provide methods, etc. for obtaining a plurality of electrical signals that propagate through a plurality of lead wires in a distinguishable form. More specifically, the objective of the present invention is to provide methods, etc. for obtaining a plurality of electrical signals that propagate through a plurality of nerves of an organism in a distinguishable form.

[Solution to Problem]

To solve the aforementioned problem, the present invention provides, for example, the following items.

(Item 1)

A method for obtaining electrical signals from a plurality of lead wires, comprising:

detecting a plurality of electrical signals from the plurality of lead wires by using an electrode structure, the electrode structure comprising a plurality of electrode layers that are disposed apart from one another, each of the plurality of electrode layers having a plurality of pores having a dimension that allows passage of the plurality of lead wires, and each of the plurality of pores being connected to a conductor for conducting an electrical signal that propagates through a passing lead wire out from the electrode structure; and
distinguishing the plurality of detected electrical signals.

(Item 2)

The method of item 1, wherein the distinguishing comprises distinguishing the plurality of electrical signals based on a propagation rate of an electrical signal, a distance between the plurality of electrode layers, and a position of a pore and a time at which an electrical signal has been detected.

(Item 3)

The method of item 1 or 2, wherein the plurality of pores have a first pore having a first dimension, and a second pore having a second dimension, which is different from the first dimension.

(Item 4)

The method of any one of items 1 to 3, wherein

the plurality of electrode layers comprise at least a first electrode layer and a second electrode layer disposed adjacent to the first electrode layer, and
a central axis of at least one of the plurality of pores in the first electrode layer is offset from a central axis of at least one of the plurality of pores in the second electrode layer.

(Item 5)

The method of item 4, wherein

the plurality of electrode layers comprise at least a first electrode layer and a second electrode layer disposed adjacent to the first electrode layer, and
a central axis of each of the plurality of pores in the first electrode layer is offset from a central axis of each of the plurality of pores in the second electrode layer.

(Item 6)

The method of item 4 or 5, wherein the plurality of electrode layers further comprise a third electrode layer disposed adjacent to the second electrode layer, the second electrode layer is disposed between the first electrode layer and the third electrode layer, and the third electrode layer has substantially the same pore arrangement as the first electrode layer.

(Item 7)

The method of item 6, wherein the plurality of electrode layers further comprise a fourth electrode layer disposed adjacent to the third electrode layer, the third electrode layer is disposed between the second electrode layer and the fourth electrode layer, and the fourth electrode layer has substantially the same pore arrangement as the second electrode layer.

(Item 8)

The method of any one of items 1 to 7, wherein the number of the plurality of electrode layers is 3 or greater, or the number of the plurality of pores is 7 or greater.

(Item 9)

The method of any one of items 1 to 8, wherein the plurality of lead wires are a plurality of nerves of an organism.

(Item 10)

An electrode structure for obtaining electrical signals from a plurality of lead wires, the electrode structure comprising a plurality of electrode layers that are disposed apart from one another, each of the plurality of electrode layers having a plurality of pores having a dimension that allows passage of the plurality of lead wires, and each of the plurality of pores being connected to a conductor for conducting an electrical signal that propagates through a passing lead wire out from the electrode structure.

(Item 11)

A system for obtaining electrical signals from a plurality of lead wires, comprising:

the electrode structure of item 10;
receiving means for receiving a plurality of electrical signals from the electrode structure; and
distinguishing means for distinguishing the plurality of electrical signals.

(Item 12)

The system of item 11, wherein the distinguishing means is configured to distinguish the plurality of electrical signals based on propagation rates of the electrical signals, a distance between the plurality of electrode layers, and positions of pores and times at which an electrical signal has been detected.

(Item 13)

A method of manufacturing an electrode structure for obtaining electrical signals from a plurality of lead wires, comprising the steps of:

preparing a plurality of electrode layers, each of the plurality of electrode layers having a plurality of pores having a dimension that allows passage of the plurality of lead wires, and each of the plurality of pores being connected to a conductor for conducting an electrical signal that propagates through a passing lead wire out from the electrode structure; and
disposing the plurality of electrode layers apart from one another.

(Item 14)

The method of manufacturing of item 13, wherein

the plurality of lead wires are a plurality of nerves of an organism, and
the step of preparing a plurality of electrode layers comprises a step of determining the number of the plurality of electrode layers and the number of the plurality of pores so that the number of trajectories the nerves can take during a process of growth of the nerves is greater than the number of the plurality of nerves.

(Item A1)

A method for obtaining electrical signals from a plurality of nerves of an organism, the method being executed by a processing unit, the method comprising:

receiving, by the processing unit, a plurality of electrical signals detected from the plurality of nerves by using an electrode structure, the electrode structure comprising a plurality of electrode layers, the plurality of electrode layers being disposed apart from one another in a direction toward which the plurality of electrode layers are overlaid, each of the plurality of electrode layers having a plurality of pores having a dimension that allows passage of the plurality of nerves, and each of the plurality of pores being connected to a conductor for conducting an electrical signal that propagates through a passing nerve out from the electrode structure; and
distinguishing, by the processing unit, one of the plurality of detected electrical signals from another one of the plurality of detected electrical signals based on positions of pores, at which the plurality of electrical signals have been detected, among the plurality of pores from another one of the plurality of detected electrical signals.

(Item A2)

The method of item A1, wherein the distinguishing comprises: calculating a time at which an electrical signal can reach each pore from a propagation rate of the electrical signal and a distance between the plurality of electrode layers; identifying nerves passing through respective pores at which the one and the another one of the plurality of electrical signals have been detected at a time matching the calculated time; and identifying that the one and the another one of the plurality of electrical signals are electrical signals obtained from the respective identified nerves to distinguish the one from the another one of the plurality of electrical signals.

(Item A3)

The method of item A1 or A2, wherein the plurality of pores have a first pore having a first dimension, and a second pore having a second dimension, which is different from the first dimension.

(Item A4)

The method of any one of items A1 to A3, wherein

the plurality of electrode layers comprise at least a first electrode layer and a second electrode layer disposed adjacent to the first electrode layer, and
a central axis of at least one of the plurality of pores in the first electrode layer is offset from a central axis of at least one of the plurality of pores in the second electrode layer.

(Item A5)

The method of item A4, wherein

the plurality of electrode layers comprise at least a first electrode layer and a second electrode layer disposed adjacent to the first electrode layer, and
a central axis of each of the plurality of pores in the first electrode layer is offset from a central axis of each of the plurality of pores in the second electrode layer.

(Item A6)

The method of item A4 or A5, wherein the plurality of electrode layers further comprise a third electrode layer disposed adjacent to the second electrode layer, the second electrode layer is disposed between the first electrode layer and the third electrode layer, and the third electrode layer has substantially the same pore arrangement as the first electrode layer.

(Item A7)

The method of item A6, wherein the plurality of electrode layers further comprise a fourth electrode layer disposed adjacent to the third electrode layer, the third electrode layer is disposed between the second electrode layer and the fourth electrode layer, and the fourth electrode layer has substantially the same pore arrangement as the second electrode layer.

(Item A8)

The method of items A1 to A7, wherein the number of the plurality of electrode layers is 3 or greater, or the number of the plurality of pores is 7 or greater.

(Item A9)

An electrode structure for obtaining electrical signals from a plurality of nerves of an organism, the electrode structure comprising a plurality of electrode layers, the plurality of electrode layers being disposed apart from one another in a direction toward which the plurality of electrode layers are overlaid, each of the plurality of electrode layers having a plurality of pores having a dimension that allows passage of the plurality of nerves, and each of the plurality of pores being connected to a conductor for conducting an electrical signal that propagates through a passing nerve out from the electrode structure.

(Item A10)

A system for obtaining electrical signals from a plurality of nerves of an organism, comprising:

the electrode structure of item A9;
receiving means for receiving a plurality of electrical signals from the electrode structure; and
distinguishing means for distinguishing one of the plurality of electrical signals from another one of the plurality of detected electrical signals based on positions of pores, at which the plurality of electrical signals have been detected, among the plurality of pores.

(Item A11)

The system of item A10, wherein the distinguishing means is configured to calculate a time at which an electrical signal can reach each pore from propagation rates of the electrical signals and a distance between the plurality of electrode layers, identify nerves passing through respective pores at which the one of the plurality of electrical signals and the another one of the plurality of electrical signals have been detected at a time matching the calculated time, and identify that the one of the plurality of electrical signals and the another one of the plurality of electrical signals are electrical signals obtained from the respective identified nerves to distinguish the one of the plurality of electrical signals from the another one of the plurality of electrical signals.

(Item A12)

The system of any of the preceding items for use in a brain-machine interface (BMI).

(Item A13)

The system of item A12, wherein

the electrode structure is embedded into a body of a subject, and
the receiving means receives a plurality of electrical signals emitted from a brain of the subject via the electrode structure embedded into the body of the subject.

(Item A14)

A method of manufacturing an electrode structure for obtaining electrical signals from a plurality of nerves of an organism, comprising the steps of:

preparing a plurality of electrode layers, each of the plurality of electrode layers having a plurality of pores having a dimension that allows passage of the plurality of nerves, and each of the plurality of pores being connected to a conductor for conducting an electrical signal that propagates through a passing nerve out from the electrode structure; and
disposing the plurality of electrode layers apart from one another in a direction toward which the plurality of electrode layers are overlaid.

(Item A15)

The method of manufacturing of item A14, wherein the step of preparing a plurality of electrode layers comprises a step of determining the number of the plurality of electrode layers and the number of the plurality of pores so that the number of trajectories a nerve can take during a process of growth of the nerve is greater than the number of the plurality of nerves.

[Advantageous Effects of Invention]

The present invention can obtain a plurality of electrical signals that propagate through a plurality of lead wires in a distinguishable form, whereby information can be extracted from the plurality of electrical signals that propagate through the plurality of lead wires. The present invention can be utilized in, for example, BMI, whereby a plurality of electrical signals that propagate through a plurality of nerves of an organism can be obtained in a distinguishable form.

[Brief Description of Drawings]

[Figure 1] Diagram showing an example of electrode layer **110** according to one embodiment of the invention.
[Figure 2] Diagram showing an example of electrode structure **100** according to one embodiment of the invention.
[Figure 3] Perspective view showing an example of an arrangement of two electrode layers in electrode structure **100** according to one embodiment of the invention.
[Figure 4] Diagram showing a specific example of two electrode layers in electrode structure **100** according to one embodiment of the invention.
[Figure 5A] Diagram showing an example of a plurality of nerves growing in electrode structure **100** according to one embodiment of the invention.
[Figure 5B] Diagram showing an example of a plurality of nerves growing in electrode structure **100** according to one embodiment of the invention.
[Figure 5C] Diagram showing an example of a plurality of nerves growing in electrode structure **100** according to one embodiment of the invention.
[Figure 5D] Diagram showing an example of a plurality of nerves growing in electrode structure **100** according to one embodiment of the invention.
[Figure 6] Diagram showing an example of a configuration of system **1000** for obtaining electrical signals from a plurality of lead wires, according to one embodiment of the invention.
[Figure 7] Flow chart showing an example of procedure **700** for obtaining electrical signals from a plurality of lead wires.
[Figure 8] Flow chart showing an example of procedure **800** for manufacturing electrical structure **100** for obtaining electrical signals from a plurality of lead wires.
[Figure 9] Diagram schematically showing an example of system **1000** for obtaining electrical signals from a plurality of lead wires according to one embodiment of the invention when used in BMI.

[Description of Embodiments]

The present invention is described hereinafter. The terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

(Definition of the terms)

As used herein, "lead wire" refers to a string-like element, which is capable of transmitting an electrical signal. In this regard, "string-like" refers to an elongated shape like a string. More specifically, this refers to a shape with the longitudinal dimension that is greater (e.g., about 5-fold or more, about 10-fold or more, about 50-fold or more, or about 100-fold or more) than the transverse dimension. A "lead wire" may be, for example, an electrically conductive member (e.g., copper wire, wire, cable, etc.) or a tissue that can transmit an electrical signal via a chemical ion (e.g., nerve of an organism, etc.).

As used herein, "organism" refers to a live organism. An organism is not limited to humans and includes, for example, animals, plants, etc.

As used herein, "about" refers to a numerical range of ± 10% from the numerical value that is described subsequent to "about".

The embodiments of the invention are described hereinafter with reference to the drawings.

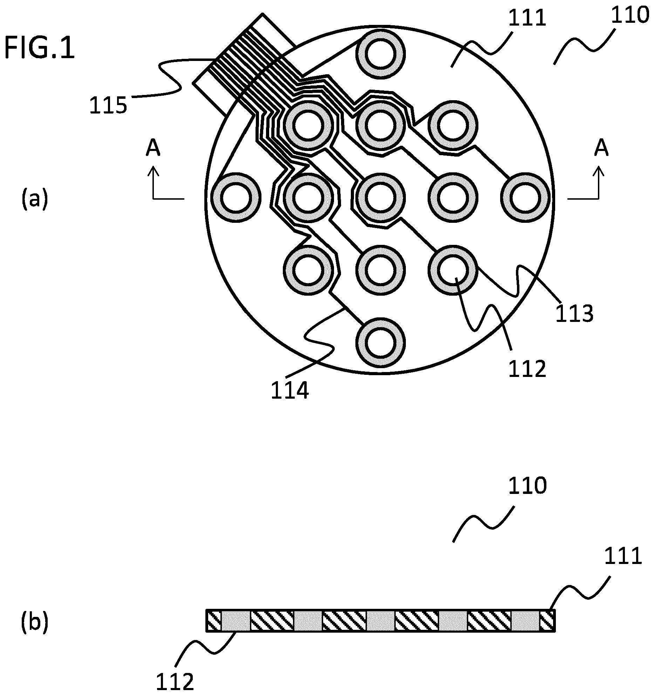

Figure 1 shows an example of an electrode layer 110 according to one embodiment of the invention. Figure 1(a) shows a plane view of the electrode layer 110. Figure 1(b) shows an A-A cross-sectional view of the electrode layer 110 along line A-A in Figure 1(a).

The electrode layer 110 comprises a substrate 111, a plurality of pores 112, and conductors 113 and 114. The electrode layer 110 can further comprise an output terminal 115.

The substrate 111 is a member that is the base of the electrode layer 110. In the example shown in Figure 1, the substrate 111 is illustrated as a substantially circular plate, but the shape of the substrate 111 is not limited thereto. The substrate 111 can have any planar shape. The substrate 111 can have a planar shape such as a substantially circular shape, substantially oval shape, substantially egg shape, substantially triangular shape, substantially quadrangular shape, or substantially polygonal shape.

The substrate 111 can have any dimension in plane view. The dimension of the substrate 111 can be a dimension in accordance with the application thereof. For example, the substrate 111 for obtaining an electrical signal from a nerve of an organism can have a dimension that fits within a square with a side of about 100,000 $\mu$m, a dimension that fits within a square with a side of about 50,000 $\mu$m, a dimension that fits within a square with a side of about 10,000 $\mu$m, a dimension that fits within a square with a side of about 5,000 $\mu$m, a dimension that fits within a square with a side of about 3,000 um, etc. More specifically, the substrate 111, when the planar shape thereof is for example substantially circular, can have a diameter of about 10,000 um or less, a diameter of about 5,000 um or less, or a diameter of about 3,000 um or less. Preferably, the substrate 111 can have a diameter of about 500 $\mu$m.

The substrate 111 can have any thickness. The thickness of the substrate 111 can be a thickness in accordance with the application thereof. For example, the substrate 111 for obtaining an electrical signal from a nerve of an organism can have a thickness of about 100 um or less, a thickness of about 50 um or less, a thickness of about 30 um or less, a thickness of about 10 um or less, etc. Preferably, the thickness of the substrate 111 can be about 20 $\mu$m.

The substrate 111 can be formed of any non-electrically-conductive material. Examples of materials for forming the substrate 111 include, but are not limited to, polyimide. Preferably, a material for forming the substrate 111 may be coated with a biocompatible material. The electrode layer 110 or an electrode structure comprised of the electrode layer 110 can be embedded into an organism by the substrate 111 being formed of a biocompatible material, and an electrical signal that propagates through a nerve of an organism can be obtained by using the electrode layer 110 or the electrode structure. Examples of the biocompatible material include, but are not limited to, MPC polymers.

A plurality of pores 112 are formed to penetrate through the substrate 111. In the example shown in Figure 1, the plurality of pores 112 are illustrated as substantially circular pores, but the shape of the plurality of pores 112 are not limited thereto. The plurality of pores 112 can have any planar shape. The plurality of pores 112 can have a planar shape such as a substantially circular shape, substantially oval shape, substantially egg shape, substantially triangular shape, substantially quadrangular shape, or substantially polygonal shape. Each of the plurality of pores 112 may have, for example, the same shape from one another, or at least one of the plurality of pores 112 may have a different shape from other pores of the plurality of pores 112.

The plurality of pores 112 can have any dimension in a plane view. The dimension of the plurality of pores 112 is a dimension in accordance with the dimension of a target lead wire from which an electrical signal is obtained. Specifically, the dimension of the plurality of pores 112 can be a dimension that allows passage of a target lead wire from which an electrical signal is obtained, whereby the electrode layer 110 can obtain an electrical signal from the lead wire passing through the pore. For example, each of the plurality of pores 112 for obtaining an electrical signal from a nerve of an organism can have a dimension that fits within a square with a side of about 1,000 μm, a dimension that fits within a square with a side of about 500 μm, a dimension that fits within a square with a side of about 300 μm, a dimension that fits within a square with a side of about 120 um, etc. More specifically, the plurality of pores **112,** when the planar shape thereof is for example substantially circular, can have a diameter of about 1000 um or less, a diameter of about 500 um or less, a diameter of about 300 um or less, or a diameter of about 120 um or less. Preferably, the plurality of pores **112** can have a diameter of about 120 um. For example, each of the plurality of pores **112** may have the same dimension from one another, or at least one of the plurality of pores **112** may have a dimension that is different from other pores of the plurality of pores **112.** In a preferred embodiment, the plurality of pores **112** can have a first pore and a second pore having a dimension that is different from that of the first pore.

The number of the plurality of pores **112** is any number that is two or greater. The number of the plurality of pores **112** is, for example, 10 or greater. Preferably, the number of the plurality of pores **112** is determined in accordance with the number of the plurality of target lead wires from which an electrical signal is obtained. For example, the number of plurality of pores **112** for obtaining an electrical signal from a nerve of an organism can be determined with the number of the plurality of electrode layers **110** in the electrode structure **100** based on the number of trajectories a nerve can take during a process of growth of the nerve as discussed below.

The interval between the plurality of pores **112** can have any value. The interval between the plurality of pores **112** is an interval in accordance with the dimension of a target lead wire from which an electrical signal is obtained. For example, the interval between the plurality of pores **112** for obtaining an electrical signal from a nerve of an organism can be, between the two closest pores, about 10 um or less, about 50 um or less, about 100 um or less, about 300 um or less, about 600 um or less, about 1,000 um or less, about 1,200 um or less, about 1,500 um or less, etc. Preferably, the interval between two pores **112** can be about 600 um or about 1200 μm.

The conductors **113** and **114** are configured to conduct an electrical signal that propagates through a lead wire passing through the plurality of pores **112** out from the electrode layer. A conductor includes a first conductor **113** disposed on the circumferential edge of the plurality of pores **112** and a second conductor **114** connected to the first conductor **113** and the output terminal **115.**

The first conductor **113** can be disposed on not only the circumferential edge of the plurality of pores **112,** but also the inner surface of the plurality of pores **112** (see Figure **1(b)**), whereby the probability of a lead wire passing through a pore contacting the first conductor **113** can be increased. The first conductor **113** may be disposed on a part of the inner surface of a pore, or the entire inner surface of a pore. The conductor **113** can be an annular ring.

In the example shown in Figure **1,** the first conductor **113** is disposed on the substrate **111** in a substantially circular shape, but the first conductor **113** can be disposed on the substrate **111** in any shape. The first conductor **113** can be disposed on the substrate **111** in a shape such as a substantially circular shape, substantially oval shape, substantially egg shape, substantially triangular shape, substantially quadrangular shape, or substantially polygonal shape.

The second conductor **114** is placed on the substrate **111** to connect the first conductor **113** with the output terminal **115.** The second conductor **114** is placed in a manner that it is electrically insulated from other first conductors **113** and other second conductors **114.** Specifically, an electrical signal from a lead wire passing through a pore can be conducted from the first conductor **113** of the pore to an output terminal without being mixed with an electrical signal from a lead wire passing through another pore, whereby an electrical signal retrieved from the output terminal **115** can be distinguished as to which pore that electrical signal has been obtained from.

The second conductor **114** connects to the first conductor **113** and the output terminal **115** through any route, as long as it is electrically insulated from other first conductors **113** and other second conductors **114.**

The conductors **113** and **114** can be formed from any material that can conduct an electrical signal. Examples of materials with which the conductors **113** and **114** are formed include, but are not limited to, stainless steel. Preferably, the material with which the conductors **113** and **114** are formed is a biocompatible material. The conductors **113** and **114** being a biocompatible material can facilitate the electrode layer **110** or an electrode structure comprised of the electrode layer **110** from being embedded into an organism. Examples of the biocompatible material include, but are not limited to, platinum.

The output terminal **115** is configured so that an electrical signal can be outputted from the electrode layer **110.** An electrical signal can be outputted out from the electrode layer **110** via the output terminal **115.**

The output terminal **115** may be configured to not only output an electrical signal, but also receive an electrical signal from the outside and communicate the received electrical signal to a corresponding pore, whereby an electrical signal can be conducted to a lead wire passing through a pore (e.g., stimulate a nerve when the lead wire is a nerve).

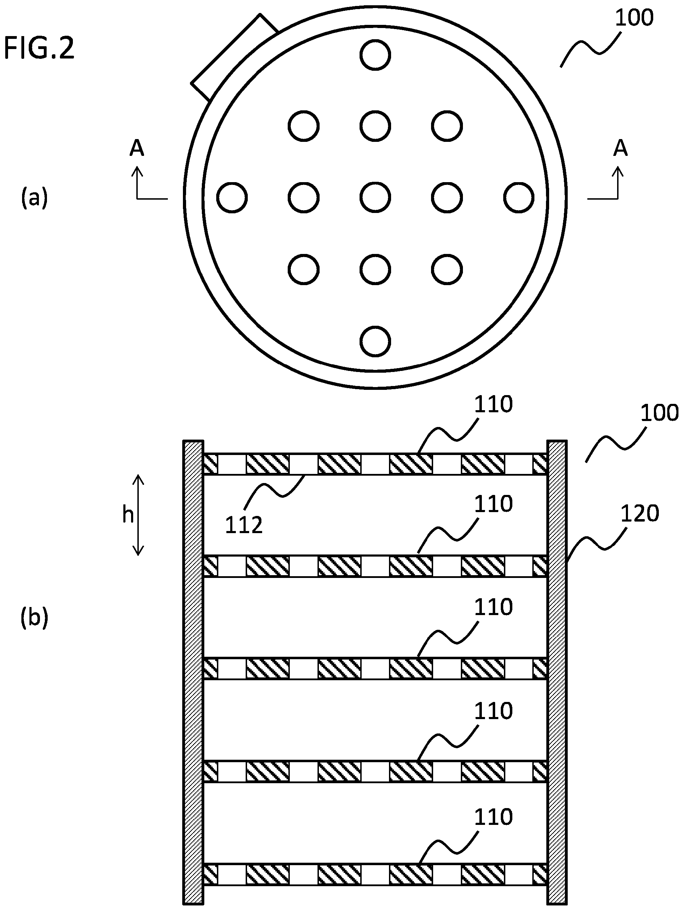

Figure **2** shows an example of the electrode structure **100** according to one embodiment of the invention. Figure **2(a)** shows a plane view of the electrode structure **100.** Figure **2(b)** shows an **A-A** cross-sectional view of the electrode structure **100** along line **A-A** in Figure **2(a).**

In the example shown in Figure **2,** the conductors **113** and **114** that the electrode layer **110** can comprise are not shown to simplify the illustration. It is understood that each of the electrode layers **110,** which the electrode structure **100** comprises, comprises the conductors **113** and **114** described with reference to Figure **1.**

The electrical structure **100** comprises a plurality of electrode layers **110.**

In the example shown in Figure **2,** the electrode structure **100** comprises five electrode layers **110,** but the electrode structure **100** can comprise any number of electrode layers **110,** which is two or greater. For example, the number of electrode layers **110** is 5 or greater. Preferably, the number of a plurality of the electrode layers **110** is determined in accordance with the number of the plurality of target lead wires from which an electrical signal is obtained. As described below, it is preferable that each of the plurality of lead wires passes through an electrode structure through a route that is unique to each of the plurality of lead wires. Thus, the number of the plurality of the electrode layers **110** and the number of the plurality of pores **112** of each electrode layer **110** are preferably the number at which each of the plurality of lead wires can follow a unique route. For example, the number of the plurality of electrode layers **110** for obtaining an electrical signal from a nerve of an organism can be determined with the number of the plurality of pores **112** of each electrode layer **110** based on the number of trajectories a nerve can take during a process of growth of the nerve.

For example, the number m of the plurality of electrode layers **110** and the number n of the plurality of pores **112** of each electrode layer can be determined as follows.

Firstly, the number fk of pores that a nerve which has passed through a pore in the k-th layer can pass through in the next k+1-th layer is represented by

[Numeral 1]

$$f_k = f_{c(ratio)}$$

wherein

[Numeral 2]

$$\text{ratio} = \frac{\frac{l}{m+1}\tan\vartheta}{r},$$
$$r = r' + d_a + d_{trace}$$

wherein θ is the maximum value of a trajectory that curves when a nerve grows, r' is the radius of a pore, l is the length of the electrode structure **100,** m is the number of layers, $d_a$ is the width of the conductor **113** (annular ring), $d_{trace}$ is the minimum distance that the conductors **113** and **114** must be separated on the substrate, and $f_{c(ratio)}$ is a function representing the number of circles that can be laid without overlapping when circles with a diameter of 1 (unit circles) are laid within a circle with a diameter = ratio.

Secondly, the number of cases after passing through all m layers is
[Numeral 3]

$$n \cdot f_2 \cdot f_3 \cdots f_m \qquad (1)$$

wherein the number of pores of the first electrode layer is n.

Thirdly, if f is assumed to have the same value in all layers to simplify the calculation, (1) can be represented by

[Numeral 4]

$$n \cdot f^{m-1}$$

The number of cases only needs to exceed the number N of a plurality of nerves of a subject, so that m and n can be selected to satisfy

[Numeral 5]

$$f > \left(\frac{\alpha \cdot N}{n}\right)^{\frac{1}{m-1}}$$

wherein α is the safety factor.

For example, calculation under the conditions of about 2800 rat sciatic nerves, safety factor of 3, and θ = 60 can result in m = 5 when n = 7 is substituted in. If a value in the actually manufacturable range is searched to a certain extent in the vicinity thereof, (m,n) = (5,7), (3,21), etc. can be obtained.

The plurality of electrode layers **110** are disposed apart at an interval **h** in the electrode structure **100.** The interval **h** can be any value. The interval **h** can be a value in accordance with the dimension of a target lead wire from which an electrical signal is obtained. The interval **h** can be, for example, a factor of the interval between a plurality of pores (e.g., about V2-fold, V3-fold, about 2-fold, about 3-fold, etc.). For example, for the electrode structure **100** for obtaining an electrical signal from a nerve of an organism, interval **h** can be about 500 um or less, about 1,000 um or less, about 1,500 um or less, about 2,000 um or less, etc. Preferably, interval **h** can be about 1,250 um or less.

In the example shown in Figure **2,** the plurality of electrode layers **110** are connected to one another by a connection member **120** that the electrode structure **100** can comprise. In the electrode structure **100,** the plurality of electrode layers **110** may be connected to one another with another member as shown in Figure **2,** or the plurality of electrode layers **110** may be integrated.

When an electrical signal is obtained from a plurality of lead wires by using the electrode structure **100,** the plurality of lead wires are in a state where the wires have passed through the electrode structure **100**. At this time, the plurality of lead wires pass through one pore of each electrode layer. Each of the plurality of lead wires preferably passes through the electrode structure via a route that is unique to each of the plurality of lead wires. Specifically, it is preferable that there are the same number of routes as the number of the plurality of lead wires. This is because when an electrical signal is obtained from a plurality of lead wires by using the electrode structure **100,** each of the plurality of lead wires having a unique route enables identification of which of the plurality of lead wires the obtained electrical signal is obtained from. Specifically, identification of which conductor **114** among the plurality of conductors **114** of the output terminal **115** of each electrode layer the detected electrical signal is obtained from enables identification of which pore the electrical signal is detected from. The unique route, and in turn the lead wire corresponding to the route, can be identified.

For example, even when electrical signals propagate simultaneously through a plurality of lead wires, it is possible to identify which lead wire among the plurality of lead wires the electrical signal is obtained from. For example, a plurality of electrical signals or noises can be identified from propagation rates of electrical signals propagating through a lead wire, a distance between electrode layers, position of pores at which an electrical signal has been detected, and times at which an electrical signal has been detected because the time at which an electrical signal can reach each pore can be calculated from the propagation rate of the electrical signal and the distance between the electrode layers, and the pore at which the electrical signal has been detected at the time matching the calculated time can be identified as the pore through which the electrical signal has passed.

In the case, for example, an electrical signal is obtained from a plurality of nerves by using the electrode structure **100,** the plurality of nerves can grow to pass through the electrode structure **100**. For example, a plurality of nerves would pass through pores of each electrode layer of the electrode structure **100** by cutting existing nerves in an organism, inserting the electrode structure **100** between the cut nerves, and then allowing the cut nerves to grow. The inventors found that: if the pores are too small, the growth of the nerves is itself inhibited such that the nerves may not successfully grow; if there are too few pores, a route unique to each of the plurality of nerves may not be able to be secured; and if the pores are too large, it would be difficult to place a conductor on a substrate and/or the size of the substrate would be too large. The inventors found a pore configuration (e.g., size, number, and arrangement of pores), which can secure an area where a conductor can be disposed on a substrate while increasing the probability of a plurality of nerves to pass through a pore without inhibiting the growth of the plurality of nerves, by suitably setting the pore configuration.

When a plurality of nerves are passed through the electrode structure **100** by allowing cut nerves to grow, an electrical signal conducted through a nerve would have two routes, i.e., the original signal route and a signal route from the electrode structure **100** to the outside. If, for example, there is a hand or a foot that is inherently controlled beyond the original signal route, an electrical signal conducted through a nerve can move the hand or foot together with an external instrument. For example, it may not be possible to move only a hand of an external instrument (e.g., robot) without moving a user's hand. For example, a dynamic clamp, etc. may be used to block the action potential at the original signal path for the prevention thereof.

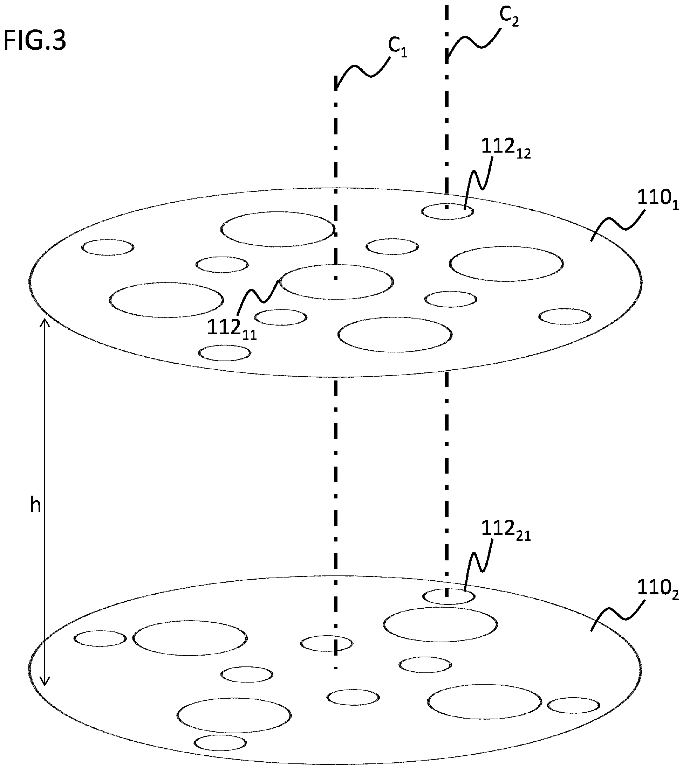

Figure **3** is a perspective view showing an example of an arrangement of two electrode layers in the electrode structure **100** according to one embodiment of the invention. The two electrode layers may be a part or all of the electrode layers that the electrode structure **100** comprises.

In the example shown in Figure **3,** the conductors **113** and **114** and the output terminal **115** that a first electrode layer $110_1$ and a second electrode layer $110_2$ can comprise are not shown to simplify the illustration. It is understood that the first electrode layer $110_1$ and second electrode layer $110_2$ comprise the conductors **113** and **114** and the output terminal **115** described in reference to Figure **1**. In the example shown in Figure **3,** the connection member **120** for connecting the first electrode layer $110_1$ and second electrode layer $110_2$ is also not shown to simplify the illustration.

The first electrode layer $110_1$ and the second electrode layer $110_2$ are disposed apart at interval **h.** Each of the first electrode layer $110_1$ and second electrode layer $110_2$ has a first pore having a first diameter and a second pore having a diameter that is smaller than the first diameter.

The first electrode layer $110_1$ and second electrode layer $110_2$ can secure an area where the conductor **113** and/or conductor **114** can be disposed on the substrate **111** while increasing the probability of a plurality of lead wires to pass through a pore by having a plurality of pores with different diameters.

Furthermore, the first electrode layer $110_1$ and the second electrode layer $110_2$ are disposed so that a central axis of at least one of the plurality of pores in the first electrode layer $110_1$ is offset from a central axis of at least one of the plurality of pores in the second electrode layer $110_2$.

For example, a first pore $112_{11}$ of the first electrode layer $110_1$ has a first central axis $C_1$, and the first central axis $C_1$ does not match the central axis of any of the plurality of pores of the second electrode layer $110_2$. Specifically, the first central axis $C_1$ is offset from the central axis of each of the plurality of pores of the second electrode layer $110_2$. In view of a central axis of a pore of the first electrode layer $110_1$ being offset from a central axis of a pore of the second electrode layer $110_2$ in this manner, a lead wire passing through a pore of the first electrode layer $110_1$ would diverge away from the direction of the central axis $C_1$ to pass through a pore of the second electrode layer $110_2$. The direction of divergence can be random. Thus, this can lead to randomization of a route a lead wire can take and in turn a route unique to a lead wire.

Meanwhile, a second pore $112_{12}$ of the first electrode layer $110_1$ has a second central axis $C_2$. The second central axis $C_2$ matches the central axis of a first pore $112_{21}$ of the second electrode layer $110_2$.

The example shown in Figure **3** describes that some of the pores of the first electrode layer $110_1$ share a central axis with some of the pores of second electrode layer $110_2$, but the present invention is not limited thereto. In a preferred embodiment, the first electrode layer $110_1$ and the second electrode layer $110_2$ may be disposed so that a central axis of each of the plurality of pores of the first electrode layer $110_1$ is offset from a central axis of each of the plurality of pores of second electrode layer $110_2$, whereby randomization of a route a plurality of lead wires can take can be promoted.

When the electrode layers shown in Figure **3** form an electrode structure for obtaining electrical signals from a plurality of lead wires, electrode layers having substantially the same pore arrangement as the first electrode layer $110_1$ and electrode layers having substantially the same pore arrangement as the second electrode layer $110_2$ can be alternatingly disposed. For example, the second electrode layer $110_2$ can be disposed adjacent to the first electrode layer $110_1$ and a third electrode layer having substantially the same pore arrangement as the first electrode layer $110_1$ can be disposed adjacent to the second electrode layer $110_2$, whereby the second electrode layer would be disposed between the first electrode layer and the third electrode layer. Furthermore, a fourth electrode layer having substantially the same pore arrangement as the second electrode layer $110_2$ can also be disposed adjacent to the third electrode layer, whereby the third electrode layer would be disposed between the second electrode layer and the fourth electrode layer. In any adjacent electrode layers in such an electrode structure, a central axis of at least one of a plurality of pores of one of the electrode layers would be offset from a central axis of at least one of the plurality of pores of the other electrode layer.

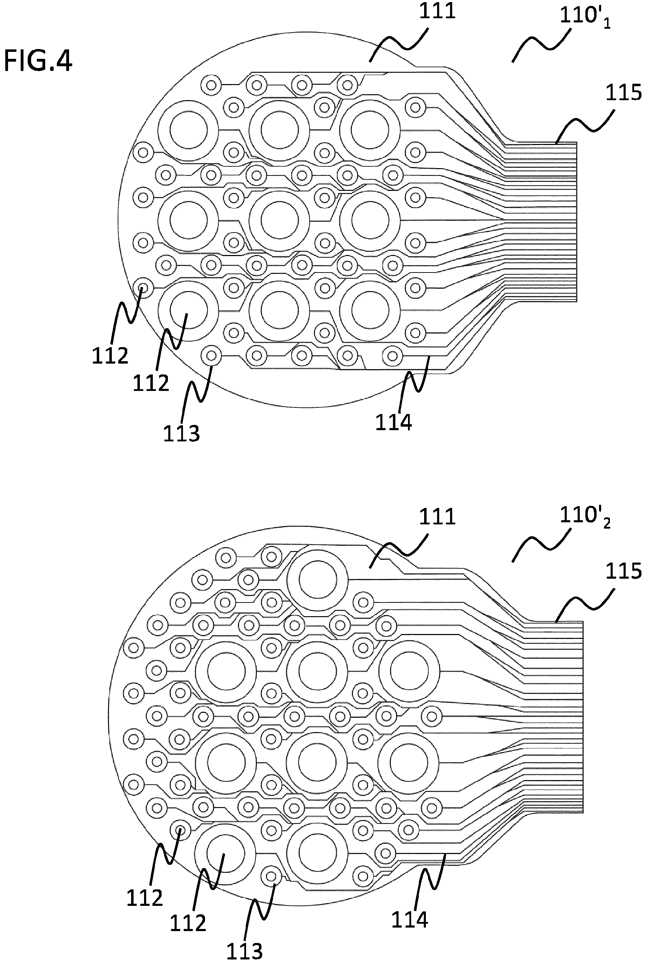

Figure **4** shows a specific example of two electrode layers in the electrode structure **100** according to one embodiment of the invention.

A first electrode layer $110'_1$ has a substantially circular substrate **111,** and has a plurality of pores **112,** a first conductor **113,** a second conductor **114,** and an output terminal **115** on the substrate **111.**

In this example, the substrate **111** has a diameter of about 5,000 um, and has an extension section for placing the second conductor **114** on the output terminal **115** side. The extension section has a dimension of about 980 $\mu$m.

The plurality of pores **112** have first pores having a first diameter and second pores having a smaller diameter than the first diameter. The first pores have a diameter of about 500 um, and the first conduct **113** is disposed around the first pores at a diameter of about 800 um. The interval between the first pores is about 1,200 um. The second pores have a diameter of about 120 um, and the first conductor **113** is disposed around the second pores at a diameter of about 270 um. The interval between the second pores is about 600 um.

A second electrode layer $110'_2$ has a substantially circular substrate **111,** and has a plurality of pores **112,** a first conductor **113,** a second conductor **114,** and an output terminal **115** on the substrate **111.**

In this example, the substrate **111** has a diameter of about 5,000 um, and has an extension section for placing the second conductor **114** on the output terminal **115** side. The extension section has a dimension of about 1,000 $\mu$m.

The plurality of pores **112** have first pores having a first diameter and second pores having a smaller diameter than the first diameter. The first pores have a diameter of about 500 um, and the first conduct **113** is disposed around the first pores at a diameter of about 800 um. The interval between the first pores is about 1,200 um. The second pores have a diameter of about 120 um, and the first conductor **113** is disposed around the second pores at a diameter of about 270 um. The interval between the second pores is about 300 $\mu$m.

The first electrode layer $110'_1$ and the second electrode layer $110'_2$ are configured so that, when overlaid on top and bottom, a central axis of each of the plurality of pores in the first electrode layer $110'_1$ is offset from a central axis of each of the plurality of pores in the second electrode layer $110'_2$. This is preferable in that randomization of routes that a plurality of lead wires can take can be promoted as described above.

The first electrode layer $110'_1$ and the second electrode layer $110'_2$ secure an area where the conductor **113** and/or conductor **114** can be disposed on the substrate **111** while increasing the probability of a plurality of lead wires to pass through a pore by having a plurality of pores with different diameters.

The electrode layers shown in Figure **4** are suitable for an electrode structure for obtaining electrical signals from a plurality of nerves of an organism. An electrode structure can be formed by disposing the electrode layers shown in Figure **4** apart from each other. In such an electrode structure, electrode layers having substantially the same pore arrangement as the first electrode layer $110'_1$ and electrode layers having substantially the same pore arrangement as the second electrode layer $110'_2$ can be, for example, alternatingly disposed. For example, the second electrode layer $110'_2$ can be dis-

posed adjacent to the first electrode layer $110'_1$ and a third electrode layer having substantially the same pore arrangement as the first electrode layer $110'_1$ can be disposed adjacent to the second electrode layer $110'_2$, whereby the second electrode layer would be disposed between the first electrode layer and the third electrode layer. Furthermore, a fourth electrode layer having substantially the same pore arrangement as the second electrode layer $110'_2$ can also be disposed adjacent to the third electrode layer, whereby the third electrode layer would be disposed between the second electrode layer and the fourth electrode layer. In any adjacent electrode layers in such an electrode structure, a central axis of each of the plurality of pores in one of the electrode layers would be offset from a central axis of each of the plurality of pores in the other electrode layer.

When an electrical signal is obtained from a plurality of lead wires by using the electrode structure **100,** the plurality of lead wires are in a state where the wires have passed through the electrode structure **100,** as described above. If the plurality of lead wires are a plurality of members with electrical conductivity such as a copper wire, wire, or cable, the plurality of members can be, for example, randomly passed through a pore of each electrode layer manually. If the plurality of lead wires are a plurality of nerves of an organism, the plurality of nerves can be, for example, allowed to randomly pass through a pore of each electrode layer via a growth process of the nerves.

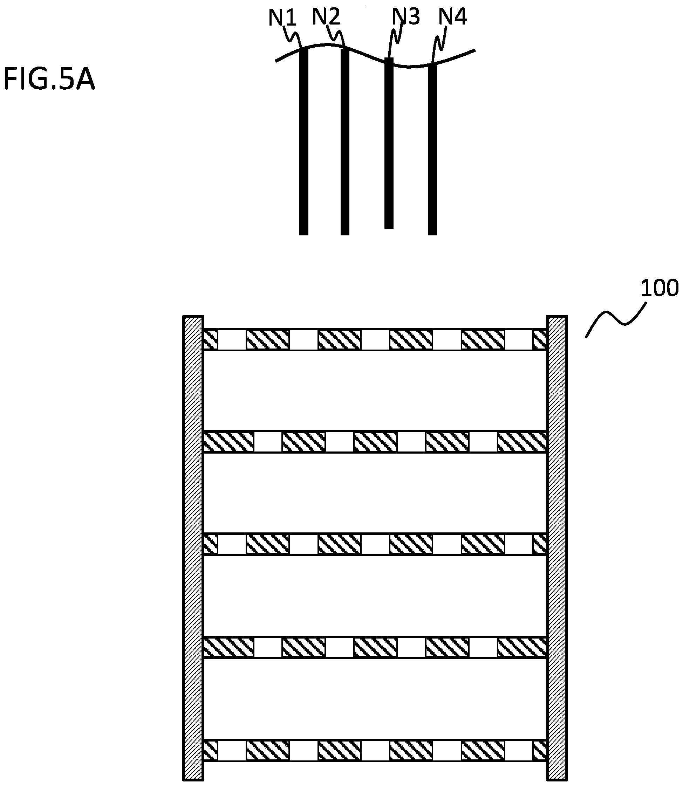

Figures **5A** to **5D** show an example of a plurality of nerves growing in the electrode structure **100** according to one embodiment of the invention.

Figures **5A** to **5D** are described using four nerves **N1** to **N4** to simplify the description. The number of plurality of nerves is not limited thereto. Any number of nerves can be used with the electrode structure **100.**

Figures **5A** to **5D** show the cross-section of the electrode layer **100.** In this example, the electrode structure **100** has five electrode layers. In the following descriptions, the electrode layers are referred to as the first electrode layer, second electrode layer, third electrode layer, fourth electrode layer, and fifth electrode layer, in order from the top. Each electrode layer has a plurality of pores. In the following descriptions, pores are referred to as the first pore, second pore, third pore ... in order from the left in each electrode layer. The m-th pore of the n-th electrode layer is referred to as $pore_{nm}$. The first electrode layer, third electrode layer, and fifth electrode layer have substantially the same electrode arrangement, and the second electrode layer and the fourth electrode layer have substantially the same electrode arrangement, whereby a central axis of each of the plurality of pores in one of adjacent electrode layers is offset from a central axis of each of the plurality of pores in the other electrode layer.

As shown in Figure **5A,** the four nerves **N1** to **N4** grow from above the electrode structure **100.**

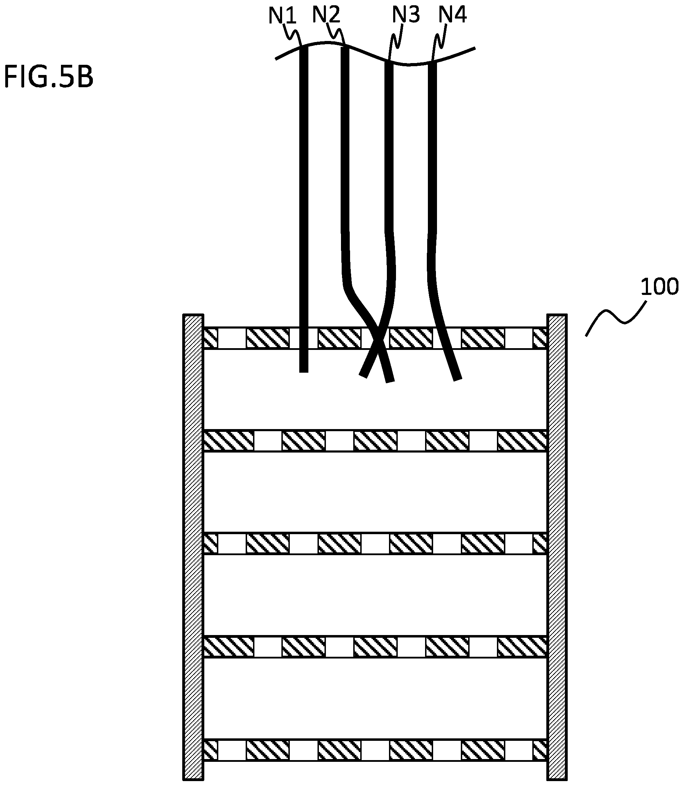

When the four nerves **N1** to **N4** grow, the nerves pass through a pore of the first electrode layer, as shown in Figure **5B.** For example, $pore_{12}$ is in the direction of growth (top to bottom direction on the page) of the first nerve **N1**. While the first nerve **N1** can continue to grow in said direction of growth, there is no pore in the direction of growth (top to bottom direction on the page) of the second nerve **N2**. Thus, the second nerve **N2** would grow by changing the direction of growth so that the nerve can pass through a pore. At this time, which direction the direction of growth of the nerve is changed to is determined in accordance with, for example, the behavioral policy of the nerve. Examples of the behavioral policy of a nerve include a policy of following other nerves or structures, etc.

In the example shown in Figure **5B,** the first nerve **N1** passes through the second $pore_{12}$ of the first electrode layer, the second nerve **N2** passes through the third $pore_{13}$ of the first electrode layer, the third nerve **N3** passes through the third $pore_{13}$ of the first electrode layer, and the fourth nerve **N4** passes through the fourth $pore_{14}$ of the first electrode layer.

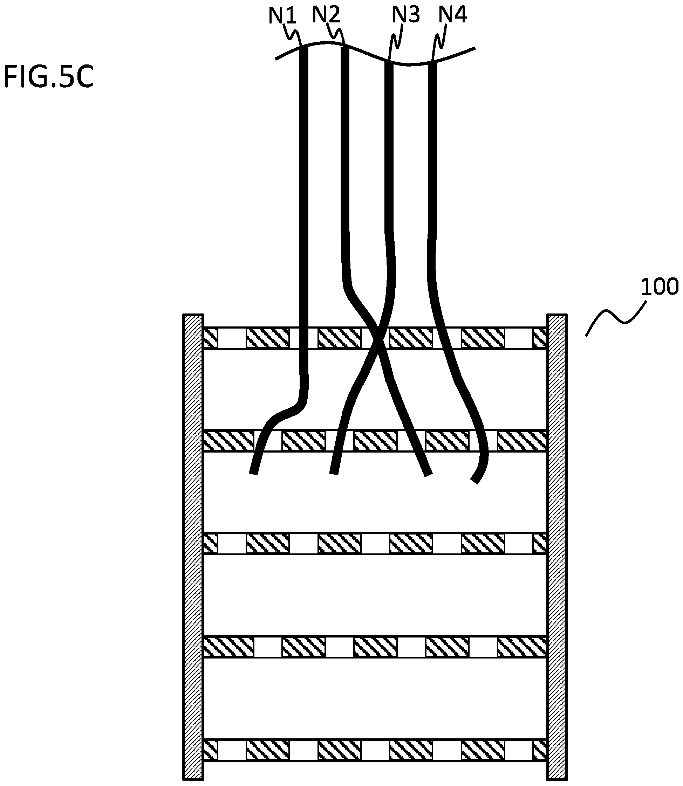

When the four nerves **N1** to **N4** grow further, the nerves would pass through a pore of the second electrode layer, as shown in Figure **5C.** Each nerve would grow by changing the direction of growth thereof so that the nerve can pass through a pore.

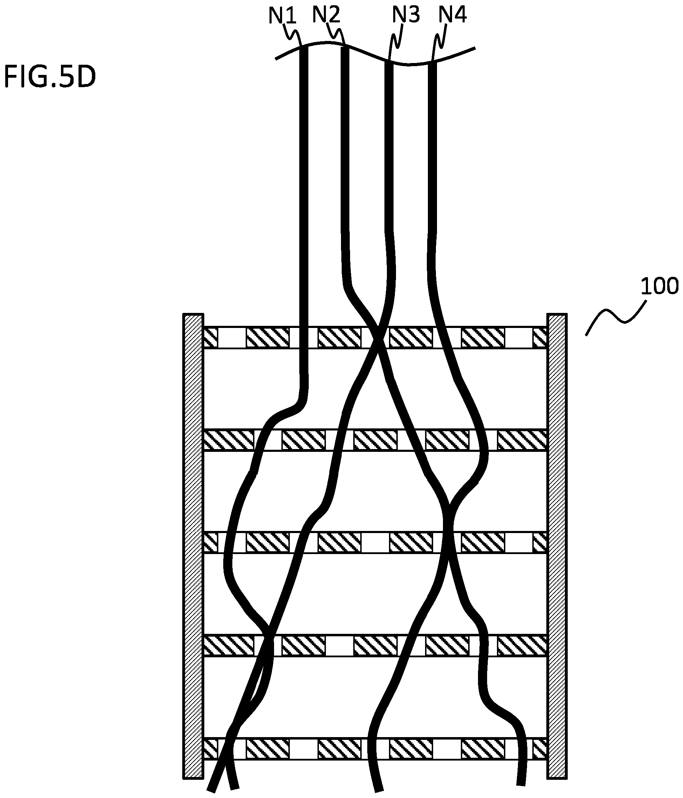

When the four nerves **N1** to **N4** grow further, the nerves would pass through pores of the third and fourth electrode layers, as shown in Figure **5D.**

In this manner, the four nerves **N1** to **N4** can pass through the electrode structure **100.** The first nerve **N1** has a route passing through $pore_{12}$-$pore_{21}$-$pore_{31}$-$pore_{41}$-$pore_{51}$. The second nerve **N2** has a route passing through $pore_{13}$-$pore_{22}$-$pore_{32}$-$pore_{41}$-$pore_{51}$. The third nerve **N3** has a route passing through $pore_{13}$-$pore_{23}$-$pore_{34}$-$pore_{43}$-$pore_{53}$. The fourth nerve **N4** has a route passing through $pore_{14}$-$pore_{24}$-$pore_{34}$-$pore_{44}$-$pore_{55}$. In this manner, the route each nerve follows is unique.

An electrical signal can be obtained from a plurality of nerves in a state where the plurality of nerves have passed through the electrode structure **100,** as shown in Figure **5D.** If, for example, an electrical signal is detected from $pore_{12}$, $pore_{21}$, $pore_{31}$, $pore_{41}$, and $pore_{51}$, the electrical signals can be identified as an electrical signal that propagates through the first nerve **N1** having a route passing through $pore_{12}$-$pore_{21}$-$pore_{31}$-$pore_{41}$-$pore_{51}$. If, for example, an electrical signal is detected from $pore_{14}$, $pore_{34}$, $pore_{34}$, $pore_{44}$, and $pore_{55}$, the electrical signals can be identified as an electrical signal that propagates through the fourth nerve **N4** having a route passing through $pore_{14}$-$pore_{24}$-$pore_{34}$-$pore_{44}$-$pore_{55}$.

For example, an electrical signal that propagates through a nerve can be distinguished from noise from the propagation rate of the electrical signal that propagates through a nerve, the distance between electrode layers, the position of a pore at which the electrical signal has been detected, and the time at which the electrical signal has been detected. If the time at which the electrical signal has been detected is significantly earlier or later than the expected time for a nerve having a certain route in view of the propagation rate of the electrical signal that propagates through the nerve, the distance between electrode layers, and the position of a pore, the electrical signal can be determined to be noise. When an electrical signal has been detected from $pore_{13}$, $pore_{33}$, $pore_{33}$, $pore_{34}$, $pore_{43}$, and $pore_{53}$ in one example, the electrical signal detected from $pore_{33}$ can be determined to be noise if the time at which the electrical signal has been detected at $pore_{33}$ is significantly earlier or later than the expected time for a nerve passing through $pore_{13}$ and $pore_{23}$ in view of the propagation rate of the electrical signal that propagates through a nerve, the distance between electrode layers, and the position of the pore. In addition, the electrical signals that have been detected at $pore_{13}$, $pore_{23}$, $pore_{34}$, $pore_{43}$, and $pore_{53}$ can be identified as an electrical signal that propagates through the second nerve **N2** having a route passing through $pore_{13}$-$pore_{23}$-$pore_{34}$-$pore_{43}$-$pore_{53}$.

For example, a plurality of electrical signals can be distinguished from the propagation rates of the electrical signals that propagate through a nerve, distance between electrode layers, positions of pores at which an electrical signal has been detected, and times at which an electrical signal has been detected. If the time at which an electrical signal has been detected is significantly earlier or later than the expected time for a nerve having a certain route in view of the propagation rate of the electrical signal that propagates through the nerve, the distance between electrode layers, and the position of a pore, the electrical signal can be determined as not originating from the nerve. When an electrical signal has been detected from $pore_{13}$, $pore_{14}$, $pore_{23}$, $pore_{24}$, $pore_{34}$, $pore_{43}$, $pore_{44}$, $pore_{53}$, and $pore_{55}$ in one example, the electrical signal detected from $pore_{44}$ and the electrical signal detected from $pore_{53}$ can be determined as having different routes if the time at which an electrical signal has been detected at $pore_{53}$ is significantly earlier or later than the expected time for a nerve passing through $pore_{44}$ in view of the propagation rate of the electrical signal that propagates through the nerve, the distance between electrode layers, and the position of a pore. If the time at which an electrical signal has been detected at $pore_{24}$ is significantly earlier or later than the expected time for a nerve passing through $pore_{13}$ in this example, the electrical signal that has been detected from $pore_{24}$ can be determined as having a different route from the electrical signal that has been detected from $pore_{13}$. In view of the above, as a possible combination of $pore_{13}$, $pore_{14}$, $pore_{23}$, $pore_{24}$, $pore_{34}$, $pore_{43}$, $pore_{44}$, $pore_{53}$, and $pore_{55}$, electrical signals that have been detected from $pore_{13}$, $pore_{23}$, $pore_{34}$, $pore_{43}$, and $pore_{53}$ can be identified as an electrical signal that propagates through the second nerve **N2** having a route passing

through $pore_{13}$-$pore_{23}$-$pore_{34}$-$pore_{43}$-$pore_{53}$ and electricals signal that have been detected from $pore_{14}$, $pore_{24}$, $pore_{34}$, $pore_{44}$, and $pore_{55}$ can be identified as an electrical signal that propagates through the fourth nerve **N4** having a route passing through $pore_{14}$-$pore_{24}$-$pore_{34}$-$pore_{44}$-$pore_{55}$. If, for example, there is no route corresponding to a route passing through a pore at which an electrical signal has been detected, the electrical signal that has been detected at the pore can be determined as noise.

If, for example, a plurality of electrical signals cannot be distinguished at a certain time, it is possible to wait for another electrical signal until an electrical signal with a different timing is detected. If an electrical signal with a different timing still cannot be detected, the electrical signals are understood to be from a plurality of redundant nerves. In such a case, the electrical signals do not need to be separated. Specifically, a plurality of electrical signals are distinguished by using the electrode structure **100** for separating the degree of freedom of control of nerves rather than for separating each nerve.

It is preferable that a plurality of electrical signals are distinguished, for example, based on electrical signals detected in multiple samplings rather than based on only an electrical signal detected in one sampling, because there can be a layer or a pore where measurement is not possible due to, for example, an issue in an electrode or an issue in sampling. If the majority of electrical signals are consistent as a whole in multiple samplings even with the presence of a layer or pore where measurement is not possible, the electrical signals can be separated from other electrical signals as effective electrical signals.

If, for example, the electrode structure **100** has a configuration that can receive an electrical signal from the outside and communicate the received electrical signal to a pore within the electrode structure in one embodiment, stimulation can be applied to a nerve passing through a pore. At this time, the route the nerve passes through can be identified by applying stimulation to a pore and detecting propagation of the stimulation at pores of each layer. This enables, for example, route the nerve passes through to be identified, and in turn an electrical signal that propagates through the nerve passing through the route to be distinguished from other electrical signals, even when an electrical signal with a different timing is not detected as described above.

In this manner, a plurality of electrical signals can be obtained from a plurality of lead wires by allowing a plurality of lead wires (e.g., plurality of nerves) to pass through the electrode structure **100.**

Obtaining a plurality of electrical signals from a plurality of lead wires can be materialized by, for example, system **1000** described below.

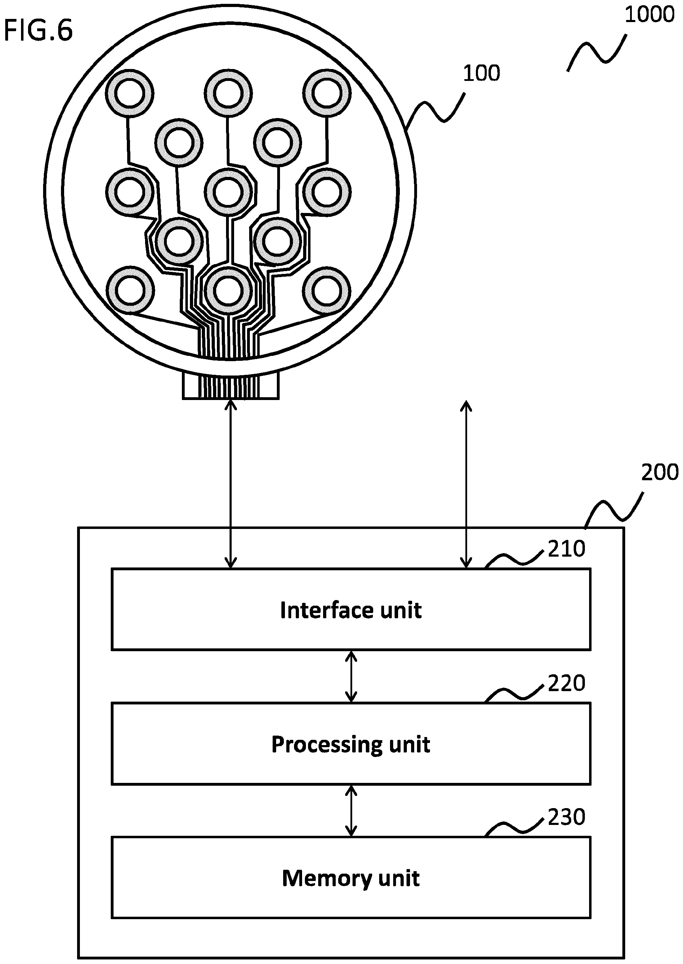

Figure **6** shows an example of a configuration of the system **1000** for obtaining electrical signals from a plurality of lead wires, according to one embodiment of the invention.

The system **1000** comprises the electrode structure **100** and a controller **200**.

The description of the electrode structure **100** is omitted because the electrode structure has the same configuration as the electrode structure **100** describes above. One electrode structure **100** is shown in the example in Figure **6**, but the number of electrode structures **100** the system **1000** comprises is not limited thereto. The system **1000** can comprise at least one electrode structure **100**.

If, for example, a plurality of electrode structures **100** are embedded into an organism and used to obtain a plurality of electrical signals from a plurality of nerves of the organism, the plurality of the electrode structures **100** may be embedded into, for example, the same organism or different organisms. If the plurality of electrode structures **100** are embedded into the same organism, the plurality of electrode structures **100** may be embedded into, for example, the same site or different sites.

The controller **200** comprises an interface unit **210**, a processing unit **220**, and a memory unit **230**.

The interface unit **210** exchanges information with an element external to the controller **200**. The processing unit **220** of the controller **200** can receive information from an element external to the controller **200** and transmit information to an element external to the controller **200**, via the interface unit **210**. The interface unit **210** can exchange information in any form.

For example, the interface unit **210** can receive an electrical signal from the electrode structure **100**. Specifically, the interface unit **210** functions as receiving means for receiving an electrical signal from the electrode structure **100**. The interface unit **210** may be configured to amplify a received signal or to receive an amplified signal.

The interface unit **210** can transmit any output based on an electrical signal received from the electrode structure **100** out of the controller **200**. An output can be utilized in any application. For example, an output can be utilized for analyzing a signal flowing within a signal line without cutting the signal line. The interface unit **210** can also transmit an electrical signal to the electrode structure **100**, whereby the interface unit **210** can establish a two-way communication with the electrode structure **100**.

The interface unit **210** can transmit a control signal to an object connected to the controller **200**. In this regard, the control signal can be generated in the processing unit **220** based on an electrical signal received from the electrode structure **100**.

An object can be, for example, an external instrument or a biological tissue. An external instrument may be, for example, a body assisting apparatus such as a prosthetic arm or a prosthetic leg, a remotely controllable robot, etc. A biological tissue may be a tissue of an organism into which the electrode structure **100** is embedded, or a tissue of an organism that is different from the organism into which the electrode structure **100** is embedded. The same electrode structure as the electrode structure **100** can be embedded into another organism. A biological tissue can be controlled by transmitting a control signal to the biological tissue. For example, a paralyzed leg can be controlled to move by transmitting a control signal based on the intent of ambulation detected by the electrode structure **100** to the paralyzed leg.

The processing unit **220** executes processing of the controller **200** and controls the overall movement of the controller **200**. The processing unit **220** reads out a program stored in the memory unit **230** and executes the program, whereby the controller **200** can function as a system for executing desired steps. The processing unit **220** may be implemented by a single processor or a plurality of processors.

The processing unit **220** can be configured to, for example, distinguish a plurality of electrical signals received via the interface unit **210**. Specifically, the processing unit **220** can function as distinguishing means for distinguishing a plurality of electrical signals.

The processing unit **220** can, for example, identify which pore of each electrode layer of the electrode structure **100** that one of the plurality of electrical signals has been detected from to identify which of the plurality of lead wires said one of the plurality of electrical signals propagates through, whereby the processing unit **220** can distinguish said one of the plurality of electrical signals from other electrical signals of the plurality of electrical signals.

The processing unit **220** can distinguish a plurality of electrical signals and/or noise based on, for example, the propagation rate of an electrical signal that propagates through a lead wire, distance between electrode layers, position of a pore at which an electrical signal has been detected, and time at which an electrical signal has been detected.

The processing unit **220** can, for example, determine that an electrical signal is not an electrical signal of a route (e.g., electrical signal of another route or noise) if the time at which the electrical signal has been detected is significantly earlier or later than the expected time for a lead wire having a certain route in view of the propagation rate of the electrical signal that propagates through the lead wire, the distance between electrode layers, and the position of a pore at which the electrical signal has been detected (e.g., earlier or later by about 1.1-fold or more, about 1.2-fold or more, about 1.5-fold or more, about 2-fold or more, etc. than the expected time).

If, for example, the processing unit **220** cannot distinguish a plurality of electrical signals at a certain time, the processing unit can wait for another electrical signal until an electrical signal with a different timing is detected to distinguish the plurality of electrical signals by using the electrical signal with a different timing.

It is preferable that a plurality of electrical signals are distinguished, for example, based on electrical signals detected in multiple samplings rather than based on only an electrical signal detected in one sampling, because there can be a layer or a pore where measurement is not possible due to, for example, an issue in an electrode or an issue in sampling. If the majority of electrical signals are consistent as a whole in multiple samplings even with the presence of a layer or pore where measurement is not possible, the electrical signals can be separated from other electrical signals as effective electrical signals by the processing unit **220.**

In one exemplary embodiment, the processing unit **220** can transmit an electrical signal to a lead wire passing through a pore via the interface unit **210** and detect the propagation of the electrical signal at a pore of each layer to identify a route through which the lead wire passes. This enables, for example, the route the lead wire passes through, and in turn an electrical signal that propagates through the route, to be distinguished from other electrical signals, even when an electrical signal with a different timing is not detected.

In one exemplary embodiment, the processing unit **220** may be configured to distinguish a plurality of electrical signals by matching a pattern with patterns of signals detected from the entire electrode structure **100.** For example, the processing unit **220** can distinguish a plurality of electrical signals by learning an electrical signal detection pattern upon passage through the electrode structure **100** in advance for each of the plurality of electrical signals that propagate through a plurality of lead wires and determining whether an electrical signal detection pattern obtained upon practice matches any of the learned electrical signal detection patterns.

The memory unit **230** stores a program required for the execution of processing of the controller **200,** data that is required for the execution of the program, etc. The memory unit **230** may store a program for processing to distinguish a plurality of electrical signals. In this regard, a program is stored in the memory unit **230** in any manner. For example, a program may be pre-installed in the memory unit **230.** Alternatively, a program may be installed into the memory unit **230** by download through a network, which can be connected to via the interface unit **210.** In such a case, the network can be of any type. The memory unit **230** can be implemented by any storage means.

In the example shown in Figure **6,** each constituent element of the controller **200** is provided within the controller **200,** but the present invention is not limited thereto. Any of the constituent elements of the controller **200** can be provided external to the controller **200.** If, for example, each of the processing unit **220** and the memory unit **230** is comprised of separate hardware parts, each hardware member may be connected via any network. At this time, the network may be of any time. Each hardware member may be connected via, for example, a LAN, a wireless connection, or a wired connection. The controller **200** is not limited to a specific hardware configuration. The processing unit **220** comprised of an analog circuit instead of a digital circuit is also within the scope of the invention. The configuration of the controller **200** is not limited to those described above, as long as the function thereof can be materialized.

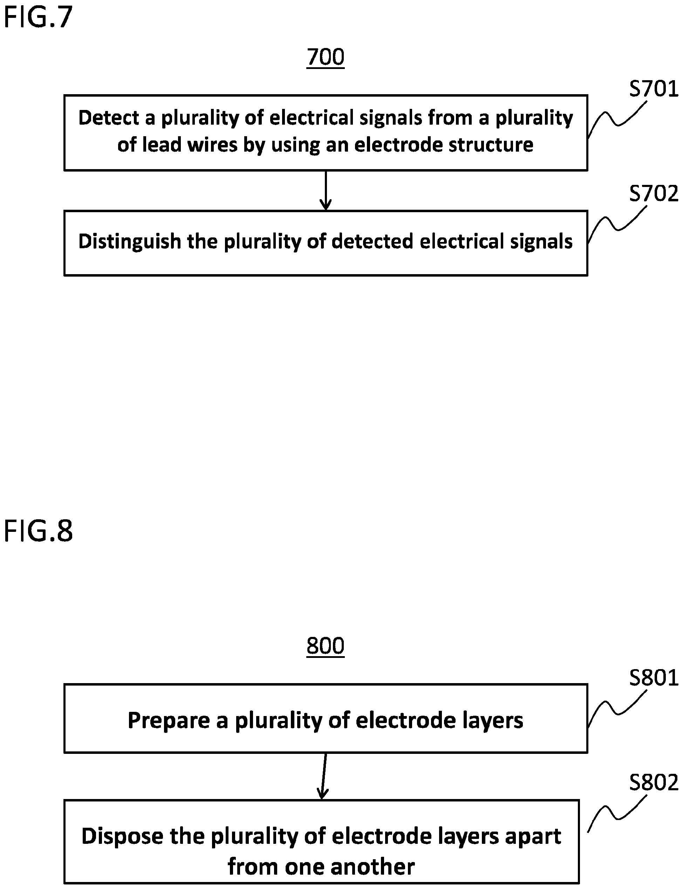

Figure **7** shows an example of procedure **700** for obtaining electrical signals from a plurality of lead wires. The procedure **700** is performed by using, for example, the system **1000** described above.

At step **S701,** a plurality of electrical signals are detected from a plurality of lead wires by using the electrode structure **100** of the system **1000.**

As described above, the electrode structure **100** comprises a plurality of electrode layers disposed apart from one another, and each of the plurality of electrode layers has a plurality of pores having a dimension that allows passage of a plurality of lead wires. Each of the plurality of pores is connected to a conductor for conducting an electrical signal that propagates through a passing lead wire out from the electrode structure.

Step **S701** is performed while a plurality of conductors are passed through the electrode structure **100.** At this time, a route the plurality of conductors follows within the electrode structure **100** is a route unique to each of the plurality of conductors. Thus, the electrode structure **100** should have the number of electrode layers and/or number of pores within each electrode layer that can form a significantly greater number of routes than the number of the plurality of conductors in order to allow a unique route for each of the plurality of conductors.

At step **S702,** the controller **200** of the system **1000** distinguishes the plurality of electrical signals detected at step **S701.**

At step **S702,** the interface unit **210** of the controller **200** first receives a plurality of electrical signals from the electrode structure **100.** The interface unit **210** can receive a plurality of electrical signals from, for example, the output terminal **115** of the electrode structure **100.** The processing unit **220** of the controller **200** then distinguishes the plurality of received electrical signals.

For example, the processing unit **220** can identify which pore of each electrode layer of the electrode structure **100** one of the plurality of electrical signals has been detected from to identify which of the plurality of lead wires said one of the plurality of electrical signals propagates through, whereby the processing unit **220** can distinguish said one of the plurality of electrical signals from other electrical signals of the plurality of electrical signals.

The processing unit **220** can distinguish a plurality of electrical signals and/or noise based on, for example, the propagation rates of the electrical signals that propagate through a lead wire, distance between electrode layers, positions of pores at which an electrical signal has been detected, and time at which an electrical signal has been detected.

The processing unit **220** can, for example, determine that an electrical signal is not an electrical signal of a route (e.g., electrical signal of another route or noise) if the time at which the electrical signal has been detected is significantly earlier or later than the expected time for a lead wire having a certain route in view of the propagation rate of the electrical signal that propagates through the lead wire, the distance between electrode layers, and the position of a pore at which the electrical signal has been detected (e.g., earlier or later by about 1.1-fold or more, about 1.2-fold or more, about 1.5-fold or more, about 2-fold or more, etc. than the expected time).

If, for example, the processing unit **220** cannot distinguish a plurality of electrical signals at a certain time, the processing unit can wait for another electrical signal until an electrical signal with a different timing is detected to distinguish a plurality of electrical signals by using the electrical signal with a different timing.

It is preferable that a plurality of electrical signals are distinguished, for example, based on electrical signals detected in multiple samplings rather than based on only an electrical signal detected in one sampling, because there can be a layer or a pore where measurement is not possible due to, for example, an issue in an electrode or an issue in sampling. If the majority of electrical signals are consistent as a whole in multiple samplings even with the presence of a layer or pore where measurement is not possible, the electrical signals can be separated from other electrical signals as effective electrical signals by the processing unit **220.**

In one exemplary embodiment, the processing unit **220** can transmit an electrical signal to a lead wire passing through a pore via the interface unit **210** and detect the propagation of the electrical signal at a pore of each layer to identify a route through which the lead wire passes. This enables, for example, the route the lead wire passes through to be identified, and in turn an electrical signal that propagates through the route to be distinguished from other electrical signals, even when an electrical signal with a different timing is not detected.

In one exemplary embodiment, the processing unit **220** may be configured to distinguish a plurality of electrical signals by matching a pattern with patterns of signals detected from the entire electrode structure **100.** For example, the processing unit **220** can distinguish a plurality of electrical signals by learning an electrical signal detection pattern upon passage through the electrode structure **100** in advance for each of the plurality of electrical signals that propagate through a plurality of lead wires and determining whether an electrical signal detection pattern obtained upon practice matches any of the learned electrical signal detection patterns.

A plurality of electrical signals can be obtained in a distinguishable form from a plurality of conductors by the procedure **700.** The obtained electrical signals can be utilized in any application. For example, a control signal for controlling an object (e.g., external instrument or biological tissue) can be generated based on an electrical signal. Since a plurality of conductors can also be distinguished, a predefined electrical signal can be conducted to a desired conductor. This enables, for example, transmission of a predefined electrical signal to a desired nerve (e.g., applying electrical stimulation, for controlling to move a paralyzed leg, to a nerve responsible for leg movement).

Figure **8** shows an example of procedure **800** for manufacturing the electrical structure **100** for obtaining electrical signals from a plurality of lead wires.

At step **S801,** a plurality of electrode layers **110** are prepared.

Each of the plurality of electrode layers **110** has a plurality of pores having a dimension that allows passage of a plurality of lead wires, as described above. Each of the plurality of pores is connected to a conductor for conducting an electrical signal that propagates through a passing lead wire out from the electrode structure.

The plurality of electrode layers **110** can be manufactured, for example, by etching, in the same manner as generic electronic substrates. The electrode layer **110** can be made by sandwiching the layers with a polyimide film, etc. in the same manner as a flexible substrate, plating an exposed electrode section as needed, etc.

At step **S802,** the plurality of electrode layers **110** prepared in step **S801** are disposed apart from one another.

The plurality of electrode layers **110** are, for example, disposed apart from one another and connected together with the connection member **120** to form the electrode structure **100.**

For example, the plurality of electrode layers **110** can be integrally formed to form the electrode structure **100.** In such a case, step **S801** and step **S802** can be performed simultaneously.

The electrode structure **100** is manufactured by the procedure **800.** The electrode structure **100** and the system **1000** comprising the electrode structure **100** can be utilized in any application as described above. The electrode structure **100** and the system **1000** comprising the electrode structure **100** can be utilized in, for example, BMI.

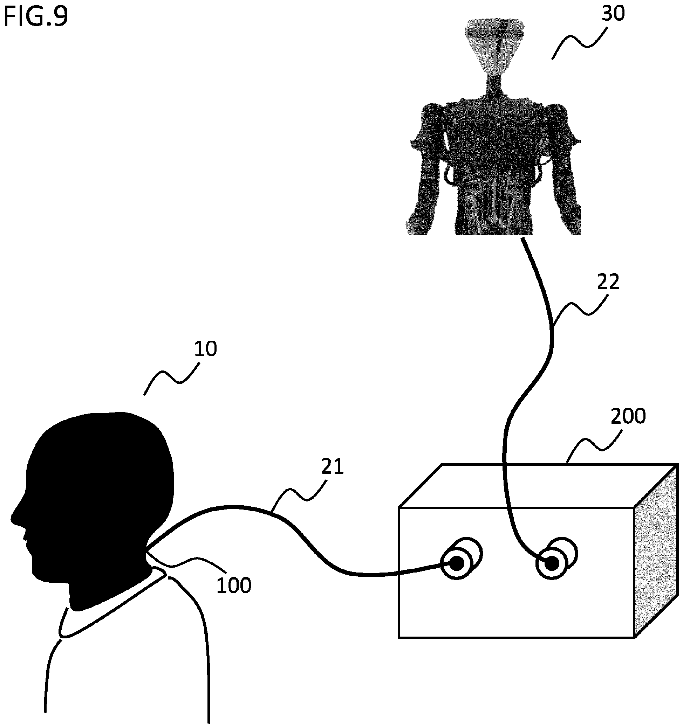

Figure **9** schematically shows an example of the system **1000** for obtaining electrical signals from a plurality of lead wires according to one embodiment of the invention when used in BMI.

This example describes an example of driving a robot **30** by using an electrical signal obtained directly from the brain of a subject **10** by the electrode structure **100.**

The electrode structure **100** is embedded into the body of the subject **10.** For example, the electrode structure **100** can be embedded into the spinal cord of the subject **10** or a nerve extending therefrom to the periphery. After the electrode structure **100** is embedded, a nerve extending from the brain is grown into the electrode structure **100,** whereby a signal that propagates in a nerve from the brain via the electrode structure **100** propagates into the electrode structure **100.**

The electrode structure **100** is connected to the controller **200** via a cable **21.** The controller **200** is connected to the robot **30** via a cable **22.** In the example shown in Figure **9,** the connection between the electrode structure and the controller **200** and the connection between the controller **200** and the robot **30** are shown as a wired connection, but a wireless connection can be used in place of the wired connection.

(Learning stage)

The subject **10** imagines operating the robot **30.** For example, the subject imagines causing the robot **30** to walk, causing the robot **30** to grasp an object, causing the robot **30** to drop the grasped object, etc. A corresponding electrical signal is emitted from the brain of the subject **10** by the subject **10** imagining operating the robot **30.**

An electrical signal emitted from the brain of the subject **10** when the subject **10** imagines each operation of the robot **30** is obtained via the electrode structure **100.** The obtained electrical signal is inputted into the controller **200** via the cable **21.** The subject **10** can input what operation was imagined into the controller **200,** whereby each operation intended by the subject is associated with the emitted signal and learned.

(Operation stage)

The subject **10** imagines operating the robot **30.** For example, the subject imagines causing the robot **30** to walk, causing the robot **30** to grasp an object, causing the robot **30** to drop the grasped object, etc. A corresponding electrical signal is emitted from the brain of the subject **10** by the subject **10** imagining operating the robot **30.**

An electrical signal emitted from the brain of the subject **10** when the subject **10** imagines each operation of the robot **30** is obtained via the electrode structure **100.** The obtained electrical signal is inputted into the controller **200** via the cable **21.** The controller **200** determines what operation was intended by the input electrical signal, when the electrical signal is emitted, by referring to the learned information, whereby the controller **200** recognizes the intent of the subject **10** from the obtained electrical signal and transmits a control signal to the robot **30** so that the robot would move as intended.

The robot **30** moves in accordance with the received control signal.

In this manner, the subject **10** can operate the robot **30** simply by imaging operating the robot **30.** The robot **30** can be moved as if the robot is the subject **10**'s own body.

The application in BMI described above is one example. The system **1000** for obtaining electrical signals from a plurality of lead wires can also be utilized in other applications.

The present invention is not limited to the embodiments described above. It is understood that the scope of the present invention should be interpreted based solely on the claims. It is understood that an equivalent scope can be practiced based on the descriptions of the present invention and common general knowledge from the specific descriptions in the preferred embodiments of the invention.

[Industrial Applicability]

The present invention is useful for providing a method for obtaining a plurality of electrical signals that propagate through a plurality of lead wires in a distinguishable form, etc.

[Reference Signs List]

    **100** electrode structure
    **110** electrode layer
    **111** substrate
    **112** pore
    **113, 114** conductor
    **115** output terminal
    **120** connection section
    **200** controller
    **1000** system

**Claims**

1. A method for obtaining electrical signals from a plurality of lead wires, comprising:

       detecting a plurality of electrical signals from the plurality of lead wires by using an electrode structure, the electrode structure comprising a plurality of electrode layers that are disposed apart from one another, each of the plurality of electrode layers having a plurality of pores having a dimension that allows passage of the plurality of lead wires, and each of the plurality of pores being connected to a conductor for conducting an electrical signal that propagates through a passing lead wire out from the electrode structure; and
       distinguishing the plurality of detected electrical signals.

2. The method of claim 1, wherein the distinguishing comprises distinguishing the plurality of electrical signals based on a propagation rate of an electrical signal, a distance between the plurality of electrode layers, and a position of a pore and a time at which an electrical signal has been detected.

3. The method of claim 1 or 2, wherein the plurality of pores have a first pore having a first dimension, and a second pore having a second dimension, which is different from the first dimension.

**4.** The method of any one of claims 1 to 3, wherein

the plurality of electrode layers comprise at least a first electrode layer and a second electrode layer disposed adjacent to the first electrode layer, and

a central axis of at least one of the plurality of pores in the first electrode layer is offset from a central axis of at least one of the plurality of pores in the second electrode layer.

**5.** The method of claim 4, wherein

the plurality of electrode layers comprise at least a first electrode layer and a second electrode layer disposed adjacent to the first electrode layer, and

a central axis of each of the plurality of pores in the first electrode layer is offset from a central axis of each of the plurality of pores in the second electrode layer.

**6.** The method of claim 4 or 5, wherein the plurality of electrode layers further comprise a third electrode layer disposed adjacent to the second electrode layer, the second electrode layer is disposed between the first electrode layer and the third electrode layer, and the third electrode layer has substantially the same pore arrangement as the first electrode layer.

**7.** The method of claim 6, wherein the plurality of electrode layers further comprise a fourth electrode layer disposed adjacent to the third electrode layer, the third electrode layer is disposed between the second electrode layer and the fourth electrode layer, and the fourth electrode layer has substantially the same pore arrangement as the second electrode layer.

**8.** The method of any one of claims 1 to 7, wherein the number of the plurality of electrode layers is 3 or greater, or the number of the plurality of pores is 7 or greater.

**9.** The method of any one of claims 1 to 8, wherein the plurality of lead wires are a plurality of nerves of an organism.

**10.** An electrode structure for obtaining electrical signals from a plurality of lead wires, the electrode structure comprising a plurality of electrode layers that are disposed apart from one another, each of the plurality of electrode layers having a plurality of pores having a dimension that allows passage of the plurality of lead wires, and each of the plurality of pores being connected to a conductor for conducting an electrical signal that propagates through a passing lead wire out from the electrode structure.

**11.** A system for obtaining electrical signals from a plurality of lead wires, comprising:

the electrode structure of claim 10;
receiving means for receiving a plurality of electrical signals from the electrode structure; and
distinguishing means for distinguishing the plurality of electrical signals.

**12.** The system of claim 11, wherein the distinguishing means is configured to distinguish the plurality of electrical signals based on propagation rates of the electrical signals, a distance between the plurality of electrode layers, and positions of pores and times at which an electrical signal has been detected.

**13.** The system of claim 11 or 12 for use in a brain-machine interface (BMI).

**14.** A method of manufacturing an electrode structure for obtaining electrical signals from a plurality of lead wires, comprising the steps of:

preparing a plurality of electrode layers, each of the plurality of electrode layers having a plurality of pores having a dimension that allows passage of the plurality of lead wires, and each of the plurality of pores being connected to a conductor for conducting an electrical signal that propagates through a passing lead wire out from the electrode structure; and
disposing the plurality of electrode layers apart from one another.

**15.** The method of manufacturing of claim 14, wherein

the plurality of lead wires are a plurality of nerves of an organism, and
the step of preparing a plurality of electrode layers comprises a step of determining the number of the plurality of electrode layers and the number of the plurality of pores so that the number of trajectories the nerves can take during a process of growth of the nerves is greater than the number of the plurality of nerves.

FIG.1

(a)

115

111    110

A        A

113

112

114

(b)

110

111

112

FIG.2

(a)

100

A    A

(b)

110
100
112
110
120
110
110
110
110

h

FIG.3

FIG.4

111    110'₁

115

112

112

113    114

111    110'₂

115

112

112

113    114

FIG.5A

FIG.5B

FIG.5C

FIG.5D

N1  N2  N3  N4

100

FIG.6

1000

100

200

210

**Interface unit**

220

**Processing unit**

230

**Memory unit**

FIG.7

700

Detect a plurality of electrical signals from a plurality of lead wires by using an electrode structure — S701

Distinguish the plurality of detected electrical signals — S702

FIG.8

800

Prepare a plurality of electrode layers — S801

Dispose the plurality of electrode layers apart from one another — S802

FIG.9

## INTERNATIONAL SEARCH REPORT

International application No.

**PCT/JP2022/016906**

### A. CLASSIFICATION OF SUBJECT MATTER

*A61B 5/294*(2021.01)i; *A61B 5/388*(2021.01)i; *G06F 3/01*(2006.01)i
FI:   A61B5/294; A61B5/388; G06F3/01 515

According to International Patent Classification (IPC) or to both national classification and IPC

### B. FIELDS SEARCHED

Minimum documentation searched (classification system followed by classification symbols)

A61B5/25-5/297; A61B5/311-5/315; A61B5/388-5/398; G06F3/01; A61F2/72; A61H1/02; B25J11/00; B25J13/00-13/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | JP 2019-4915 A (UNIV. OF ELECTRO-COMMUNICATIONS) 17 January 2019 (2019-01-17) <br>    paragraphs [0017]-[0044], fig. 1-8 | 1-15 |
| A | JP 2005-389 A (NATIONAL INST. OF ADVANCED INDUSTRIAL & TECHNOLOGY) 06 January 2005 (2005-01-06) <br>    paragraphs [0014]-[0040], fig. 1-9 | 1-15 |
| A | JP 61-217136 A (AGCY. OF IND. SCIENCE & TECHNOL.) 26 September 1986 (1986-09-26) <br>    page 2, lower left column, line 18 to page 4, upper left column, line 5, fig. 1-4 | 1-15 |
| A | JP 61-217137 A (AGCY. OF IND. SCIENCE & TECHNOL.) 26 September 1986 (1986-09-26) <br>    page 2, lower left column, line 6 to page 3, lower left column, line 5, fig. 1-2 | 1-15 |
| A | JP 9-24031 A (AGCY. OF IND. SCIENCE & TECHNOL.) 28 January 1997 (1997-01-28) <br>    paragraphs [0005]-[0012], fig. 1-5 | 1-15 |

☑ Further documents are listed in the continuation of Box C.      ☑ See patent family annex.

| | |
|---|---|
| *    Special categories of cited documents: <br> "A"  document defining the general state of the art which is not considered to be of particular relevance <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search | Date of mailing of the international search report |
|---|---|
| **07 June 2022** | **21 June 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
| --- |
| **PCT/JP2022/016906** |

### C. DOCUMENTS CONSIDERED TO BE RELEVANT

| Category* | Citation of document, with indication, where appropriate, of the relevant passages | Relevant to claim No. |
| --- | --- | --- |
| A | 吉田充宏, 廣瀬義人, 浮田芳昭, 満渕邦彦, 内海裕一, 多層配線可能な神経再生型電極の作製, エレクトロニクス実装学術講演大会講演論文集, 2011, vol. 25, 10C-13, 2011, pp. 345-346, (YOSHIDA, Mitsuhiro, HIROSE, Yoshihito, UKITA, Yoshiaki, MABUCHI, Kunihiko, UTSUMI, Yuichi. Proposal of stacked electrodes for multiplex neural interface. Proceedings of JIEP Annual Meeting.)<br>entire text, all drawings | 1-15 |

Form PCT/ISA/210 (second sheet) (January 2015)

**INTERNATIONAL SEARCH REPORT**
Information on patent family members

International application No.

**PCT/JP2022/016906**

| Patent document cited in search report | | | Publication date (day/month/year) | Patent family member(s) | Publication date (day/month/year) |
|---|---|---|---|---|---|
| JP | 2019-4915 | A | 17 January 2019 | WO 2017/073770 A1 paragraphs [0023]-[0067], fig. 1-8 | |
| JP | 2005-389 | A | 06 January 2005 | (Family: none) | |
| JP | 61-217136 | A | 26 September 1986 | (Family: none) | |
| JP | 61-217137 | A | 26 September 1986 | (Family: none) | |
| JP | 9-24031 | A | 28 January 1997 | US 5692516 A column 2, line 6 to column 4, line 13, fig. 1-5 EP 0753284 A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018147407 A